## Europäisches Patentamt

## European Patent Office

## Office européen des brevets

(19)

(11) Veröffentlichungsnummer: **0 101 008**
**B1**

(12) **EUROPÄISCHE PATENTSCHRIFT**

(45) Veröffentlichungstag der Patentschrift:
**01.06.88**

(51) Int. Cl.⁴: **B 01 J  23/66,** B 01 J  23/96,
C 07 D  301/10

(21) Anmeldenummer: **83107641.9**

(22) Anmeldetag: **03.08.83**

(54) Verfahren zur Verbesserung der Wirksamkeit von gebrauchten Silber-Trägerkatalysatoren.

(30) Priorität: **07.08.82  DE 3229541**

(43) Veröffentlichungstag der Anmeldung:
**22.02.84 Patentblatt 84/8**

(45) Bekanntmachung des Hinweises auf die Patenterteilung:
**01.06.88 Patentblatt 88/22**

(84) Benannte Vertragsstaaten:
**BE DE FR GB IT NL SE**

(56) Entgegenhaltungen:
**EP - A - 0 001 078
EP - A - 0 025 963
DE - A - 2 940 480
FR - A - 2 369 275
GB - A - 1 591 308
GB - A - 2 045 636
US - A - 4 125 480**

(73) Patentinhaber: **HOECHST AKTIENGESELLSCHAFT,
Postfach 80 03 20, D-6230 Frankfurt am Main 80 (DE)**

(72) Erfinder: **Rebsdat, Siegfried, Dr., Trenbeckstrasse 24,
D-8261 Burg, Neuötting (DE)**
Erfinder: **Mayer, Sigmund, Hochfellnstrasse 20,
D-8261 Burgkirchen (DE)**
Erfinder: **Alfranseder, Josef, Hauptstrasse 31,
D-8261 Hofschallern, Post Marktl (DE)**

## Beschreibung

Die Erfindung betrifft ein Verfahren zur Verbesserung der Wirksamkeit von gebrauchten Silber-Trägerkatalysatoren für die Herstellung von Ethylenoxid durch Umsetzung von Ethylen mit molekularem Sauerstoff oder Luft, bei dem der gebrauchte Katalysator mit einer Lösung aus Wasser, einem wassermischbaren organischen Lösungsmittel, ausgewählt aus der Gruppe bestehend aus Methanol, Ethanol, Propanol und Isopropanol, und mindestens einer Cäsiumverbindung, Rubidiumverbindung oder Cäsium- und Rubidiumverbindung in einer Menge von 50 bis 1 000 mg Cäsium, Rubidium oder Cäsium und Rubidium pro Kilogramm Lösung imprägniert wird, die überschüssige Imprägnierlösung abgetrennt und der imprägnierte Katalysator getrocknet wird.

Zur Herstellung von Ethylenoxid durch Oxidation von Ethylen mit Sauerstoff oder Luft werden Silberkatalysatoren eingesetzt, deren Herstellung seit langem bekannt und in verschiedenen Patentschriften beschrieben ist. Eine ganze Reihe grosstechnischer Anlagen zur Herstellung von Ethylenoxid arbeiten nach dem Silberkatalysator-Verfahren.

Dabei wird üblicherweise nur ein Bruchteil des eingesetzten Ethylens umgesetzt. Das umgesetzte Ethylen wird an dem mit Silber imprägnierten Trägermaterial mit Sauerstoff zum überwiegenden Teil in Ethylenoxid überführt, der Rest wird praktisch vollständig in Kohlendioxid und Wasser verwandelt.

Im Laufe der Zeit sind die verschiedensten Silberkatalysatoren entwickelt worden, und zwar mit dem Ziel, die Selektivität in bezug auf die bevorzugte Bildung von Ethylenoxid zu erhöhen und die Bildung von $CO_2$ und Wasser zurückzudrängen.

Bei steigenden Rohstoffpreisen und zunehmender Rohstoffverknappung kommt einer erhöhten Selektivität der Katalysatoren besondere wirtschaftliche Bedeutung zu. In den letzten Jahren sind in der Literatur im Prinzip zwei Wege beschrieben worden, mit denen Silber-Trägerkatalysatoren mit erhöhter Selektivität erhalten werden können. Der eine Weg beruht auf der Entwicklung neuer Silber-Trägerkatalysatoren, die sich von den älteren insbesondere durch eine spezielle Morphologie des aufgebrachten Silbers, durch ein spezielles Trägermaterial oder durch ausgewählte Promotoren unterscheiden.

Beispielsweise ist in der DE-A-2 300 512 ein Silber-Trägerkatalysator beschrieben, der dadurch erhalten wird, dass man auf Aluminiumoxid gleichzeitig mit dem Silber 0,0004 bis 0,0027 g-Äquivalente einer Kalium-, Rubidium- oder Cäsiumverbindung pro Kilogramm Katalysator aus wässriger Lösung aufbringt.

Der andere Weg zur Herstellung von Silber-Trägerkatalysatoren mit erhöhter Selektivität beruht darauf, dass bei einem an sich fertigen Katalysator durch eine Nachbehandlung die Selektivität wesentlich verbessert wird. Dabei wird von einem Silber-Trägerkatalysator ausgegangen, der bereits zur Produktion von Ethylenoxid mehr oder weniger lang benutzt worden ist. Die Nachbehandlung besteht darin, dass der gebrauchte Katalysator mit einer Lösung bestehend aus Wasser, einem wassermischbaren organischen Lösungsmittel und mindestens einer Cäsium- und/oder Rubidiumverbindung in einer Menge von 50 bis 1000 mg, vorzugsweise 80 bis 500 mg, insbesondere 100 bis 400 mg Cäsium und/oder Rubidium pro Kilogramm Lösung imprägniert wird, die überschüssige Imprägnierlösung abgetrennt und der imprägnierte Katalysator getrocknet wird. Der so behandelte Katalysator wird nun wieder zur Herstellung von Ethylenoxid durch Umsetzung von Ethylen mit molekularem Sauerstoff oder Luft eingesetzt.

Solche Verfahren zur Verbesserung der Wirksamkeit von gebrauchten Silber-Trägerkatalysatoren sind in DE-C-2 519 599, DE-C-2 611 856, DE-C-2 636 680 und DE-C-2 649 359 und in DE-A-2 712 785, DE-A-2 740 480, DE-A-2 746 976, DE-A-2 751 767, DE-A-2 820 520 und DE-A-2 938 245 beschrieben.

Bei allen diesen bekannten Verfahren zur Selektivitätsverbesserung von gebrauchten Silberkatalysatoren wird der Temperatur, bei der der gebrauchte Katalysator mit der Imprägnierlösung (Regenerierlösung) behandelt wird, keinerlei Bedeutung beigemessen. Die Behandlung wird fast immer bei Raumtemperatur durchgeführt. Lediglich beim Verfahren der DE-A-2 938 245 wird bei einer Temperatur bis zu 30 °C imprägniert (vergleiche Beispiele). Die Imprägniertemperatur selbst wird aber auch hier im Hinblick auf die Selektivitätsverbesserung offensichtlich als bedeutungslos angesehen.

Auch die Imprägnierzeit (Einwirkungszeit), das ist die Zeit, in welcher der zu imprägnierende Katalysator mit der Imprägnierflüssigkeit in Kontakt bleibt, wird keine besondere Bedeutung beigemessen. Sie wird lediglich nach praktischen Erwägungen ausgerichtet. So werden, falls überhaupt Zeitangaben gemacht werden, Einwirkungszeiten von 3 bis 120 Minuten, vorzugsweise 5 bis 20 Minuten genannt (vergleiche DE-C-2 636 680 und DE-C-2 649 359 sowie DE-A-2 740 480).

Die Menge an Wasser in der Imprägnierlösung wird ebenfalls als bedeutungslos für den Erfolg der Nachbehandlung eines gebrauchten Silber-Trägerkatalysators angesehen. Der Wassermenge wird offensichtlich lediglich die Aufgabe eines Lösungsvermittlers für die eingesetzte Cäsium- und Rubidiumverbindung in dem wassermischbaren organischen Lösungsmittel zugeschrieben. Zum Beispiel ist die Löslichkeit einiger Cäsiumverbindungen in reinem Methanol so niedrig, dass unter Umständen die erforderliche Cäsiumkonzentration in der Imprägnierlösung nur schwer zu erreichen ist. Lediglich aus diesem Grund wird eine Wassermenge von bis zu 40 Gew.-% in der Imprägnierlösung genommen (vergleiche zum Beispiel DE-C-2 636 680 und DE-C-2 649 359 sowie DE-A-2 712 785, Seite 11).

Bei einigen der bekannten Verfahren zur Reaktivierung von gebrauchten Silber-Trägerkatalysatoren wird zur Erreichung des gewünschten Erfolges empfohlen, der Cäsium- und/oder Rubidiumverbindungen enthaltenden Imprägnierlösung spezielle Sauerstoff- und/oder Stickstoffverbindungen zuzusetzen (vergleiche DE-A-2 751 767 und DE-A-2 820 520) oder den Katalysator vor der Imprägnierung zu waschen (vergleiche DE-A-2 740 480, DE-A-2 746 976 und DE-A-2 938 245).

Zu den bisher abgehandelten Druckschriften sind, insbesondere bezüglich Imprägniertemperatur, noch GB-A-1 591 308 und DE-A-2 940 480 zu erwähnen. In der britischen Patentschrift wird eine Imprägniertemperatur von 20 bis 150 °C genannt. Nachdem aber in allen Beispielen bei Raumtemperatur imprägniert wird und auch in der Beschreibung keine weiteren Ausführungen zu dem angegebenen Temperaturbereich gemacht werden, wird einer Imprägnierung bei einer höheren Temperatur als der Raumtemperatur offensichtlich keinerlei Bedeutung beigemessen. In der genannten deutschen Offenlegungsschrift wird als Temperatur bei der Imprägnierung des gebrauchten Silber-Trägerkatalysators mit einer Imprägnierlösung aus einem Erdalkali- und einem Alkalimetall und niederen Alkanolen sowie gegebenenfalls Wasser als Lösungsmittel der Bereich von 10 bis 50 °C, vorzugsweise 20 bis 30 °C, genannt. Auch hier wird in allen Beispielen bei Raumtemperatur imprägniert, weil höhere Imprägniertemperaturen offensichtlich als bedeutungslos angesehen werden.

Nach dem Stand der Technik über die Verbesserung der Wirksamkeit von gebrauchten Silber-Trägerkatalysatoren wird also weder der Imprägniertemperatur noch der Imprägnierzeit und dem Wassergehalt der Imprägnierlösung eine kritische Bedeutung beigemessen. Um beim Imprägnieren mit Cäsium- und/oder Rubidiumverbindungen enthaltenden Lösungen eine höhere Selektivitätssteigerung zu erreichen, wird vielmehr häufig der Einsatz von zusätzlichen speziellen Verbindungen in der Imprägnierlösung oder eine Wäschebehandlung des gebrauchten Katalysators empfohlen. Diese Massnahmen sind umständlich und zeitaufwendig. Im Falle des Waschens ist sogar ein zusätzlicher Verfahrensschritt erforderlich.

Es wurde nun überraschenderweise gefunden, dass bei der bekannten Reaktivierung von gebrauchten Silber-Trägerkatalysatoren durch Behandlung mit einer Imprägnierlösung aus Wasser, einem wassermischbaren organischen Lösungsmittel aus niederen Alkanolen und mindestens einer Cäsiumverbindung, Rubidiumverbindung oder Cäsium- und Rubidiumverbindung in einer Menge von 50 bis 1000 mg, vorzugsweise 80 bis 500 mg, insbesondere 100 bis 400 mg Cäsium, Rubidium oder Cäsium und Rubidium pro Kilogramm Lösung, die Imprägniertemperatur, die Imprägnierzeit und die Menge an Wasser in der Imprägnierlösung für die Erreichung eines hohen Reaktivierungsgrades kritisch ist, und dass eine hohe Selektivitätsverbesserung erreicht werden kann, wenn bei erhöhter Temperatur imprägniert wird, die Imprägnierzeit 0,75 bis 10 Stunden beträgt und die Imprägnierlösung einen Wassergehalt von 5 bis 50 Gew.-%, bezogen auf das Gewicht der Lösung, enthält.

Das eingangs angegebene Verfahren zur Verbesserung der Wirksamkeit von gebrauchten Silber-Trägerkatalysatoren für die Herstellung von Ethylenoxid durch Umsetzung von Ethylen mit molekularem Sauerstoff oder Luft ist demnach erfindungsgemäss dadurch gekennzeichnet, dass bei einer Temperatur von 80 bis 150 °C imprägniert wird, die Imprägnierzeit 0,75 bis 10 Stunden beträgt und die Imprägnierlösung 5 bis 50 Gew.-% Wasser, bezogen auf das Gewicht der Lösung, enthält.

Die Imprägniertemperatur beträgt vorzugsweise 90 bis 130 °C.

Der Wassergehalt der Imprägnierlösung beträgt vorzugsweise 10 bis 40 Gew.-%, bezogen auf das Gewicht der Lösung.

Das Herstellen der Imprägnierlösung, das Imprägnieren des gebrauchten Silberkatalysators, das Abtrennen der überschüssigen Imprägnierflüssigkeit nach dem Imprägnieren und das Trocknen des imprägnierten Katalysators ist aus den oben angegebenen Druckschriften bekannt. So erhält man die zu verwendende Imprägnierlösung in einfacher Weise durch Mischen der einzelnen Bestandteile.

Geeignete Cäsium- und Rubidiumverbindungen sind die (wasserlöslichen) Salze und Hydroxide des Cäsiums und Rubidiums.

Zweckmässige Salze (anorganische oder organische) sind die Nitrate, Carbonate, Bicarbonate, Formiate, Acetate, Oxalate und Lactate, wobei aus rein praktischen Gründen die Nitrate und Acetate bevorzugt sind.

Die Art der Cäsium- und Rubidiumverbindung ist für den erfindungsgemässen Erfolg nicht entscheidend. Ebenso bedeutungslos ist das Anion im Cäsium- und Rubidiumsalz. Man kann eine oder mehrere Cäsium- oder Rubidiumverbindungen einsetzen, Mischungen von Cäsium- und Rubidiumverbindungen sind ebenfalls geeignet.

Eine bevorzugte Imprägnierlösung besteht aus 10 bis 40 Gew.-% Wasser, 80 bis 500 mg pro Kilogramm Lösung Cäsium und/oder Rubidium in Form einer (wasserlöslichen) Cäsium- und Rubidiumverbindung, vorzugsweise in Form eines (wasserlöslichen) Salzes oder Hydroxids des Cäsiums und Rubidiums, und aus einem oder mehreren Alkanolen mit 1 bis 3 C-Atomen als Ergänzung (Rest) auf 100 Gewichtsprozent Lösung.

Besonders bevorzugt wird eine Imprägnierlösung eingesetzt, die aus 10 bis 40 Gew.-% Wasser, 100 bis 400 mg pro Kilogramm Lösung Cäsium und/oder Rubidium in Form einer (wasserlöslichen) Cäsium- und Rubidiumverbindung, vorzugsweise in Form eines (wasserlöslichen) Salzes oder Hydroxids des Cäsiums und Rubidiums, und aus einem oder mehreren Alkanolen mit 1 bis 3 C-Atomen als Ergänzung auf 100 Gewichtsprozent Lösung besteht.

Die Menge an Imprägnierlösung wird im allgemeinen so gewählt, dass ein volumenmässiger Überschuss, bezogen auf den zu imprägnierenden Silberkatalysator vorliegt. Im allgemeinen wird das 0,5- bis 3fache, vorzugsweise 1- bis 2fache Volumen an Imprägnierflüssigkeit, bezogen auf das Volumen an zu imprägnierendem Katalysator, genommen.

Nach einer zweckmässigen Methode wird die Imprägnierung in der Weise durchgeführt, dass der Katalysator in einem Behälter (Autoklaven) mit der Imprägnierlösung übergossen wird und Katalysator und Imprägnierflüssigkeit, gegebenenfalls unter Rühren, auf die erfindungsgemässe Temperatur erhitzt und bei dieser Temperatur während der erfindungsgemässen Zeit gehalten werden.

Nach einer anderen zweckmässigen Methode, dem sogenannten Fluten, wird die Imprägnierlösung an dem in einem oder mehreren Rohren als Festbett angeordneten Katalysator durchgeführt. Diese Methode empfiehlt sich besonders bei Grossanlagen, in denen der zu regenerierende Katalysator bereits in den Rohren des Reaktors vorliegt. Das Übergiessen (Fluten) kann einmal oder mehrmals (mit der abgetrennten oder mit einer neu bereiteten Imprägnierflüssigkeit), kontinuierlich oder diskontinuierlich durchgeführt werden. Beim kontinuierlichen Imprägnieren wird die Imprägnierlösung unter Einhaltung der erfindungsgemässen Imprägnierzeit im Kreis gepumpt, beim diskontinuierlichen wird sie in den Rohren durch deren Verschliessung der erfindungsgemässen Zeit entsprechend lang gehalten. Auch im Fall des Flutens ist es zweckmässig, die erfindungsgemässe Imprägniertemperatur dadurch zu erreichen und einzustellen, dass Katalysator und Imprägnierflüssigkeit auf diese Temperatur erhitzt werden.

Es versteht sich von selbst, dass bei der erfindungsgemässen Imprägnierung aufgrund der erhöhten Imprägniertemperatur Drucke auftreten, die den Dampfdrücken der eingesetzten Imprägnierflüssigkeiten entsprechen.

Nach der Imprägnierung wird die überschüssige Imprägnierflüssigkeit vom Katalysator abgetrennt, durch Abgiessen, Abfiltrieren, Abzentrifugieren oder im Falle des Flutens einfach durch Abfliessenlassen.

Nachdem die überschüssige Imprägnierflüssigkeit abgetrennt ist, wird der imprägnierte, noch mehr oder weniger feuchte Katalysator getrocknet. Die Trocknung erfolgt im allgemeinen bei einer Temperatur von 20 bis 150 °C, vorzugsweise 50 bis 120 °C. Die Abdampfung des Lösungsmittels kann beispielsweise mit Hilfe von Hordentrocknern, Drehrohröfen oder durch Überleiten von heissen Inertgasen wie Stickstoff, Luft und/oder Kohlendioxid, durchgeführt werden. Die Temperatur richtet sich im allgemeinen nach dem Siedepunkt des Lösungsmittels der Imprägnierflüssigkeit. Nach einer bevorzugten Arbeitsweise wird die Trocknung in Gegenwart von Inertgas und bei einer Temperatur von 50 bis 120 °C durchgeführt.

Nach der Trocknung liegt der fertige, in der Wirksamkeit verbesserte Katalysator vor.

Das erfindungsgemässe Verfahren ist unabhängig von der Art des Silber-Trägerkatalysators selbst. Jeder für die Direktoxidation von Ethylen zu Ethylenoxid mit molekularem Sauerstoff oder Luft geeignete und gebrauchte Silberkatalysator kommt für das erfindungsgemässe Verfahren in Betracht.

Silberkatalysatoren für die Direktoxidation von Ethylen zu Ethylenoxid mit molekularem Sauerstoff oder Luft sind, ebenso wie die Direktoxidation selbst, in der Literatur eingehend beschrieben, beispielsweise in US-A-2 615 899, US-A-3 899 445 und US-A-3 962 136. Die in Rede stehenden Silberkatalysatoren bestehen in der Regel aus Silber in einer Menge von 3 bis 20 Gew.-%, vorzugsweise 7 bis 14 Gew.-%, und gegebenenfalls Promotoren wie Kalium, Natrium, Lithium, Cäsium und/oder Rubidium, im allgemeinen in einer Menge von 0,002 bis 0,08 Gew.-%, vorzugsweise 0,003 bis 0,05 Gew.-%, auf einem hitzebeständigen, porösen Trägermaterial (Gewichtsprozente jeweils bezogen auf den gesamten Katalysator). Das Silber befindet sich bekanntlich als Metall auf den inneren und äusseren Oberflächen des Trägermaterials. Die Morphologie des auf dem Trägermaterial aufgebrachten Silbers kann innerhalb weiter Grenzen variieren. Im allgemeinen hat es die Gestalt von kugelförmigen Teilchen mit einem Durchmesser von 0,01 bis 10 µm. Beispiele für Trägermaterialien sind Aluminiumoxide verschiedenster Struktur, Magnesiumoxide, Kieselgur, Bimsstein, Siliciumdioxid, Siliciumcarbid, Tone, Korund, Zeolithe, Metalloxide und ähnliche. Besonders bevorzugte Trägermaterialien sind α-Aluminiumoxide, da sie einen weitgehend gleichmässigen Porendurchmesser aufweisen. Die Trägermaterialien werden in der Regel durch die spezifische Oberfläche ($m^2/g$), das spezifische Porenvolumen ($cm^3/g$) und den mittleren Porendurchmesser (µm) charakterisiert. Sie weisen im allgemeinen die Form von Granulaten, Kugeln, Ringen oder dergleichen auf.

Das erfindungsgemässe Verfahren bezieht sich auf gebrauchte Silber-Trägerkatalysatoren, das heisst auf solche Silber-Trägerkatalysatoren, die zur Umsetzung von Ethylen zu Ethylenoxid mit molekularem Sauerstoff oder Luft bereits eingesetzt waren. Die Zeit ihres Einsatzes kann innerhalb weiter Grenzen variieren; sie kann wenige Wochen bis mehrere Jahre betragen. Dabei kann der Katalysator in seiner Wirksamkeit nachgelassen (was in der Regel nach relativ langer Gebrauchszeit der Fall ist) oder die ursprüngliche Wirksamkeit praktisch beibehalten haben. Beim erfindungsgemässen Verfahren werden vorzugsweise solche gebrauchten Silber-Trägerkatalysatoren eingesetzt, deren Wirksamkeit abgesunken ist, die also zu den sogenannten gealterten oder ermüdeten Silberkatalysatoren zählen. Die gebrauchten Katalysatoren können Promotoren wie Natrium, Kalium, Lithium, Cäsium und/oder Rubidium enthalten, oder promotorfrei sein. Sie können bereits ein- oder mehrmals nach einer der bekannten Methoden regeneriert (reaktiviert) worden sein, beispielsweise nach der Methode der deutschen Patentschrift 2 611 856. Das erfindungsgemässe Verfahren ist besonders vorteilhaft bei solchen gealterten Silber-Trägerkatalysa-

toren, die mindestens 1mal, vorzugsweise 1- bis 4mal, nach einer der bekannten Methoden regeneriert worden sind und daher Cäsium und/oder Rubidium enthalten.

Nach Durchführung des erfindungsgemässen Verfahrens liegt ein Silberkatalysator vor, der in der Regel 30 bis 500 mg, vorzugsweise 40 bis 400 mg Cäsium und/oder Rubidium pro Kilogramm Katalysator enthält.

Eine Erklärung für die unerwartete Steigerung der Wirksamkeit durch das erfindungsgemässe Verfahren kann nicht gegeben werden. Offensichtlich wirken die gegenüber den bekannten Regenerierungsverfahren erhöhte Imprägniertemperatur, die Imprägnierzeit und die Imprägnierlösung miteinander zusammen. Es stellt sich offensichtlich ein besonderes Gleichgewicht zwischen der Cäsium- und/oder Rubidiumkonzentration in der Imprägnierlösung und jener auf dem zu regenerierenden Katalysator ein.

Die Wirksamkeit eines Silberkatalysators kann bekanntlich ausgedrückt werden als prozentualer Umsatz des eingesetzten Ethylens bei einer bestimmten Reaktionstemperatur und als Selektivität, das ist der molare Prozentsatz an umgesetztem Ethylen, das zu Ethylenoxid reagiert hat. Ein Katalysator ist umso wirksamer, je mehr Ethylen bei einer bestimmten Temperatur umgesetzt wird, je höher die Selektivität bei einem bestimmten Umsatz und je niedriger die Temperatur zur Erzielung eines bestimmten Umsatzes ist.

Mit dem erfindungsgemässen Verfahren wird nicht nur die Selektivität von gebrauchten Silber-Trägerkatalysatoren, sondern auch der Umsatz gesteigert. Daher ist es möglich, bei erfindungsgemäss behandelten Katalysatoren die Reaktionstemperatur abzusenken, bei gleichem oder sogar erhöhtem Umsatz. Dies ist deshalb besonders bedeutungsvoll, weil mit niedriger Reaktionstemperatur die Bildung unerwünschter Nebenprodukte wie Kohlendioxid, Formaldehyd und Acetaldehyd, stark zurückgeht. Angesichts der grossen Mengen Ethylenoxids, die nach dem Ethylen-Oxidationsverfahren produziert werden, kommt einer Ausbeutesteigerung auch nur um wenige Prozent oder sogar um Zehntelprozent erhebliche wirtschaftliche Bedeutung zu. Was das erfindungsgemässe Verfahren ferner auszeichnet, ist der Umstand, dass es in den üblichen Grossproduktionsanlagen (mit handelsüblichen Silber-Trägerkatalysatoren) ohne nennenswerten zusätzlichen Aufwand an Investitionen und Material durchgeführt werden kann.

Die Erfindung wird nun an Beispielen (erfindungsgemässen Beispielen und Vergleichsbeispielen) noch näher erläutert, wobei auch der kritische Punkt der erfindungsgemässen Verfahrensparameter, Imprägniertemperatur, Imprägnierzeit und Wassergehalt der Imprägnierlösung, bewiesen wird. Die Beispiele wurden in einem Versuchsreaktor aus einem senkrecht stehenden Reaktionsrohr aus Chromvanadiumstahl mit einer lichten Weite von 30 mm und einer Länge von 300 mm durchgeführt. Das mit einem Mantel versehene Reaktionsrohr wurde mit heissem Öl, das durch den Mantel strömte, beheizt. Das Reaktionsrohr war bis zu einer Höhe von 200 mm mit α-Al$_2$O$_3$-Pellets gefüllt; diese Füllung diente zur Vorheizung des Einsatzgases. Auf der inerten Füllung lag der zu prüfende Katalysator. Das Einsatzgas trat unten (drucklos) in das Reaktionsrohr ein und verliess es am Kopf.

Das eingesetzte Gasgemisch bestand aus

| | |
|---|---|
| C$_2$H$_4$ | 28 Vol.-% |
| CH$_4$ | 53 Vol.-% |
| O$_2$ | 8 Vol.-% |
| CO$_2$ | 5 Vol.-% |
| N$_2$ | 6 Vol.-% |
| Vinylchlorid | 0,0002 Vol.-% (Inhibitor). |

Die Raum-Zeit-Geschwindigkeit betrug:

$$250 \cdot \frac{\text{Raumteile Gas}}{\text{Stunde} \cdot \text{Raumteile Katalysator}}.$$

Die Temperatur des Wärmeträgermediums wurde solange variiert, bis ein konstanter Ethylenumsatz von 7% erzielt wurde.

Das am Reaktorausgang austretende Gas wurde gaschromatographisch analysiert und aus den Analysenergebnissen wurden der Umsatz und die Selektivität berechnet.

In den Beispielen wurden handelsübliche gebrauchte Silber-Trägerkatalysatoren verwendet (sie hatten etwa 10 Gew.-% Silber mit einem Teilchendurchmesser von 1 bis 5 μm und α-Al$_2$O$_3$ mit einer spezifischen Oberfläche von 0,1 bis 0,5 m$^2$/g als Trägermaterial).

Nachstehend sind die verwendeten Katalysatoren im einzelnen angeführt.

Katalysator I: ist ein Katalysator, der keine Alkalimetalle als Promoter enthielt. Er war 7 Jahre zur Ethylenoxidproduktion eingesetzt.

Katalysator II: er entspricht dem Katalysator I, mit dem Unterschied, dass dieser nach den 7 Jahren Einsatz mit einer Cäsiumnitrat enthaltenden Imprägnierlösung gemäss DE-C-2 611 856 regeneriert worden ist und anschliessend weitere 2 Jahre zur Ethylenoxid-Produktion eingesetzt war.

Katalysator III: ist ein Katalysator, der keine Alkalimetalle als Promotor enthielt und 4 Jahre zur Ethylenoxid-Produktion eingesetzt war.

Katalysator IV: er entspricht dem Katalysator III, mit dem Unterschied, dass dieser nach den 4 Jahren Einsatz mit einer Cäsiumnitrat und Ammoniak enthaltenden Imprägnierlösung gemäss DE-A-2 938 245 regeneriert worden ist und dann weitere 2 Jahre zur Ethylenoxid-Produktion eingesetzt war.

Katalysator V: ist ein Katalysator, der (aufgrund seiner Herstellung gemäss DE-A-2 300 512) schon von vornherein Cäsium als Promoter enthielt. Er war 2 Jahre zur Ethylenoxid-Produktion eingesetzt.

Beispiele 1 bis 30

In den Beispielen wurden die Katalysatoren I bis V mit Hilfe des beschriebenen Versuchsreaktors unter den angegebenen Bedingungen getestet.

Nachstehend sind die Beispiele 1 bis 30 im einzelnen angeführt. Die entscheidenden Bedingungen bei der Durchführung der Beispiele und die Testergebnisse sind in einer Tabelle zusammengefasst (die nicht erfindungsgemässen Beispiele sind durch den Zusatz – Vergleichsbeispiel – gekennzeichnet).

Beispiel 1 (Vergleichsbeispiel)

Der Katalysator I wurde ohne weitere Behandlung getestet.

Beispiel 2 (Vergleichsbeispiel)

Der Katalysator I wurde gemäss Beispiel 7 der DE-C-2 611 856 regeneriert und dann getestet. -

Beispiel 3

Der Katalysator I wurde erfindungsgemäss regeneriert und dann getestet.

0,513 g Cäsiumnitrat wurden in 199,5 g destilliertem Wasser gelöst. Diese Lösung wurde in 800 g Methanol (technisch) eingerührt. Die erhaltene Imprägnierlösung bestand demnach aus 20 Gew.-% Wasser, 350 mg Cäsium (in Form von Cäsiumnitrat) pro Kilogramm Lösung, das sind 0,051 Gew.-% Cäsiumnitrat, und dem organischen Lösungsmittel Methanol als Rest auf 100 Gewichtsprozent Lösung, das sind 79,949 Gew.-%.

50 g Katalysator I wurden in einem 250 ml-Autoklaven mit 40 g Imprägnierlösung übergossen. Es wurde auf 100 °C erhitzt und bei dieser Temperatur (Imprägniertemperatur) 5 Stunden lang stehengelassen. Danach wurde abgekühlt, die überschüssige Imprägnierlösung abgegossen und der noch feuchte Katalysator im Trockenschrank bei 120 °C getrocknet. Der so erfindungsgemäss behandelte (regenerierte) Katalysator I wurde nun getestet.

Beispiel 4 (Vergleichsbeispiel)

Der Katalysator II wurde ohne weitere Behandlung getestet.

Beispiel 5 (Vergleichsbeispiel)

Der Katalysator II wurde gemäss Beispiel 10 der DE-A-2 938 245 regeneriert und dann getestet.

Beispiele 6 bis 9

Der Katalysator II wurde, wie im (erfindungsgemässen) Beispiel 3 beschrieben, unter Anwendung der Imprägniertemperaturen 80 °C, 100 °C, 120 °C und 150 °C regeneriert. Jeder der erhaltenen Katalysatoren wurde getestet.

Beispiel 10

Der Katalysator II wurde, wie in Beispiel 3 beschrieben, unter Anwendung einer Cäsiumkonzentration in der Imprägnierlösung von 250 mg Cäsium pro Kilogramm Lösung behandelt und getestet.

Beispiele 11 bis 14

Der Katalysator II wurde, wie im Beispiel 3 beschrieben, unter Anwendung einer Imprägnierzeit von 1 Stunde, 3 Stunden, 6 Stunden und 10 Stunden behandelt, wobei im Beispiel 12 anstelle von 350 mg Cäsium 100 mg pro Kilogramm Imprägnierlösung vorlagen. Jeder der erhaltenen Katalysatoren wurde getestet.

Beispiel 15

Der Katalysator II wurde, wie im Beispiel 3 beschrieben, unter Anwendung von Ethanol als organischem Lösungsmittel und 150 mg Cäsium pro Kilogramm Imprägnierlösung regeneriert und getestet.

Beispiel 16

Der Katalysator II wurde, wie im Beispiel 3 beschrieben, unter Anwendung von Isopropanol als organischem Lösungsmittel und 400 mg Cäsium pro Kilogramm Imprägnierlösung, behandelt und getestet.

Beispiele 17 bis 21

Der Katalysator II wurde, wie im Beispiel 3 beschrieben, unter Anwendung eines Wassergehalts in der Imprägnierlösung von 5, 10, 20, 30 und 50 Gew.-%, bezogen auf das Gewicht der fertigen Lösung, und einer Imprägnierzeit von jeweils 3 Stunden regeneriert. Jeder der erhaltenen Katalysatoren wurde getestet.

Beispiel 22 (Vergleichsbeispiel)

Der Katalysator III wurde ohne weitere Behandlung getestet.

Beispiel 23 (Vergleichsbeispiel)

Der Katalysator III wurde gemäss Beispiel 10 der DE-A-2 938 245 regeneriert und dann getestet.

Beispiel 24

Der Katalysator III wurde wie in Beispiel 3, unter Anwendung einer Imprägnierzeit von 3 Stunden, regeneriert und getestet.

Beispiel 25 (Vergleichsbeispiel)

Der Katalysator IV wurde ohne weitere Behandlung getestet.

Beispiel 26 (Vergleichsbeispiel)

Der Katalysator IV wurde gemäss Beispiel 10 der DE-A-2 938 245 regeneriert und dann getestet.

Beispiel 27

Der Katalysator IV wurde, wie im Beispiel 3 beschrieben, unter Anwendung von 3 Stunden Imprägnierzeit, regeneriert und getestet.

Beispiel 28 (Vergleichsbeispiel)

Der Katalysator V wurde ohne weitere Behandlung getestet.

Beispiel 29

Der Katalysator V wurde, wie im Beispiel 3 beschrieben, unter Anwendung einer Imprägnierzeit von 3 Stunden regeneriert und getestet.

Beispiel 30

Der Katalysator V wurde, wie im Beispiel 3 beschrieben, unter Anwendung von Cäsiumacetat und 3 Stunden Imprägnierzeit regeneriert und getestet.

Tabelle

| Beispiel Nr. (V9-Vergleichs-beispiel) | Bedingungen bei der Regenerierung Zusammensetzung der Imprägnierlösung | | | | | | | Ergebnis der Regenerierung | |
|---|---|---|---|---|---|---|---|---|---|
| | Lösungs-mittel | Wasser-menge (Gew.-%) | Cäsium-verbindung | mg Cs pro kg Lösung | Cs-Verbin-dung in der Lösung (Gew.-%) | Tempera-tur (°C) | Zeit (h) | Tempera-tur für 7% $C_2H_4$-Umsatz | Selekti-vität bei 7% $C_2H_4$-Umsatz |
| 1 VB | | | | | | | | 260 | 68,5 |
| 2 VB | | | | | | | | 230 | 77,0 |
| 3 | $CH_3OH$ | 20 | $CsNO_3$ | 350 | 0,051 | 100 | 5 | 225 | 77,7 |
| 4 VB | | | | | | | | 242 | 72,0 |
| 5 VB | | | | | | | | 228 | 77,5 |
| 6 | $CH_3OH$ | 20 | $CsNO_3$ | 350 | 0,051 | 80 | 5 | 223 | 77,7 |
| 7 | $CH_3OH$ | 20 | $CsNO_3$ | 350 | 0,051 | 100 | 5 | 220 | 78,1 |
| 8 | $CH_3OH$ | 20 | $CsNO_3$ | 350 | 0,051 | 120 | 5 | 219 | 78,2 |
| 9 | $CH_3OH$ | 20 | $CsNO_3$ | 350 | 0,051 | 150 | 5 | 219 | 78,2 |
| 10 | $CH_3OH$ | 20 | $CsNO_3$ | 250 | 0,037 | 100 | 5 | 220 | 78,0 |
| 11 | $CH_3OH$ | 20 | $CsNO_3$ | 350 | 0,051 | 100 | 1 | 220 | 78,0 |
| 12 | $CH_3OH$ | 20 | $CsNO_3$ | 100 | 0,015 | 100 | 3 | 220 | 78,2 |
| 13 | $CH_3OH$ | 20 | $CsNO_3$ | 350 | 0,051 | 100 | 6 | 220 | 78,3 |
| 14 | $CH_3OH$ | 20 | $CsNO_3$ | 350 | 0,051 | 100 | 10 | 220 | 78,3 |
| 15 | $C_2H_5OH$ | 20 | $CsNO_3$ | 150 | 0,022 | 100 | 5 | 219 | 78,2 |
| 16 | $i\text{-}C_3H_7OH$ | 20 | $CsNO_3$ | 400 | 0,059 | 100 | 5 | 220 | 78,1 |
| 17 | $CH_3OH$ | 5 | $CsNO_3$ | 350 | 0,051 | 100 | 3 | 225 | 77,7 |
| 18 | $CH_3OH$ | 10 | $CsNO_3$ | 350 | 0,051 | 100 | 3 | 222 | 78,2 |
| 19 | $CH_3OH$ | 20 | $CsNO_3$ | 350 | 0,051 | 100 | 3 | 220 | 78,3 |
| 20 | $CH_3OH$ | 30 | $CsNO_3$ | 350 | 0,051 | 100 | 3 | 220 | 78,2 |
| 21 | $CH_3OH$ | 50 | $CsNO_3$ | 350 | 0,051 | 100 | 3 | 230 | 78,0 |
| 22 VB | | | | | | | | 260 | 68,0 |
| 23 VB | | | | | | | | 228 | 77,4 |
| 24 | $CH_3OH$ | 20 | $CsNO_3$ | 350 | 0,051 | 100 | 3 | 219 | 78,3 |
| 25 VB | | | | | | | | 245 | 73,0 |
| 26 VB | | | | | | | | 227 | 77,3 |
| 27 | $CH_3OH$ | 20 | $CsNO_3$ | 350 | 0,051 | 100 | 3 | 220 | 77,9 |
| 28 VB | | | | | | | | 235 | 75,0 |
| 29 | $CH_3OH$ | 20 | $CsNO_3$ | 350 | 0,051 | 100 | 3 | 217 | 78,2 |
| 30 | $CH_3OH$ | 20 | $CsDOCCH_3$ | 350 | 0,050 | 100 | 3 | 221 | 78,1 |

## Patentansprüche

1. Verfahren zur Verbesserung der Wirksamkeit von gebrauchten Silber-Trägerkatalysatoren für die Herstellung von Ethylenoxid durch Umsetzung von Ethylen mit molekularem Sauerstoff oder Luft, bei dem der gebrauchte Katalysator mit einer Lösung aus Wasser, einem wassermischbaren organischen Lösungsmittel, ausgewählt aus der Gruppe bestehend aus Methanol, Ethanol, Propanol und Isopropanol, und mindestens einer Cäsiumverbindung, Rubidiumverbindung oder Cäsium- und Rubidiumverbindung in einer Menge von 50 bis 1000 mg Cäsium, Rubidium oder Cäsium und Rubidium pro Kilogramm Lösung imprägniert wird, die überschüssige Imprägnierlösung abgetrennt und der imprägnierte Katalysator getrocknet wird, dadurch gekennzeichnet, dass bei einer Temperatur von 80 bis 150°C imprägniert wird, die Imprägnierzeit 0,75 bis 10 Stunden beträgt und die Imprägnierlösung 5 bis 50 Gew.-% Wasser, bezogen auf das Gewicht der Lösung, enthält.

2. Verfahren nach Anspruch 1, dadurch gekennzeichnet, dass bei einer Temperatur von 90 bis 130°C imprägniert wird.

3. Verfahren nach einem der Ansprüche 1 oder 2, dadurch gekennzeichnet, dass die Imprägnierlösung 10 bis 40 Gew.-% Wasser enthält.

## Claims

1. A process for improving the activity of used supported silver catalysts for the manufacture of ethylene oxide by reaction of ethylene with molecular oxygen or air by impregnating the used catalyst with a solution consisting of water, a water-miscible organic solvent selected from the group consisting of methanol, ethanol, propanol and isopropanol, and at least one cesium compound, rubidium compound or cesium and rubidium compound in an amount of from 50 to 1000 mg of cesium, rubidium or cesium and rubidium per kg of solution, separating the excess impregnation solution, and drying the impregnated catalyst, characterised in that the impregnation is carried out at a temperature of from 80 to 150°C, the impregnation time is from 0.75 to 10 hours, and the impregnating solution contains from 5 to 50 weight % of water, relative to the weight of the solution.

2. The process as claimed in claim 1, which comprises impregnating at a temperature of from 90 to 130°C.

3. The process as claimed in claim 1, wherein the impregnating solution contains from 10 to 40 weight % of water.

## Revendications

1. Procédé pour améliorer l'efficacité, après qu'ils ont été utilisés, de catalyseurs à base d'argent sur support qui servent à préparer l'oxyde d'éthylène par réaction de l'éthylène avec l'oxygène moléculaire ou l'air, procédé selon lequel le catalyseur usé est imprégné avec une solution constituée d'eau, d'un solvant organique, miscible à l'eau, choisi dans l'ensemble constitué par le méthanol, l'éthanol, le propanol et l'isopropanol, et d'une quantité d'au moins un composé du césium, un composé du rubidium ou un composé du césium et du rubidium qui correspond à une quantité de césium, de rubidium, ou de césium et de rubidium, de 50 à 1000 mg par kilogramme de la solution, l'excès de la solution d'imprégnation est éliminé, et le catalyseur imprégné est séché, et qui est caractérisé en ce que l'imprégnation est effectuée à une température de 80 à 150°C, la durée de l'imprégnation est de 0,75 à 10 heures, et la solution d'imprégnation contient de 5 à 50% en poids d'eau par rapport au poids de la solution.

2. Procédé selon la revendication 1, caractérisé en ce qu'on effectue l'imprégnation à une température de 90 à 130°C.

3. Procédé selon l'une des revendications 1 et 2, caractérisé en ce que la solution d'imprégnation contient de 10 à 40% en poids d'eau.